(19)

Europäisches
Patentamt
European
Patent Office
Office européen
des brevets

(11) **EP 1 797 426 B1**

(12) **EUROPEAN PATENT SPECIFICATION**

(45) Date of publication and mention
of the grant of the patent:
**06.01.2010  Bulletin 2010/01**

(21) Application number: **05794452.2**

(22) Date of filing: **21.09.2005**

(51) Int Cl.:
***G01N 33/53*** *(2006.01)*        ***G01N 33/80*** *(2006.01)*
***H03H 9/19*** *(2006.01)*

(86) International application number:
**PCT/IT2005/000539**

(87) International publication number:
**WO 2006/033130 (30.03.2006 Gazette 2006/13)**

(54) **RAPID MONITORING SYSTEM FOR BLOOD GROUPS AND IMMUNOHEMATOLOGICAL REACTION DETECTION**

SCHNELLES ÜBERWACHUNGSSYSTEM FÜR BLUTGRUPPEN UND IMMUNHÄMATOLOGISCHER REAKTIONSNACHWEIS

SYSTEME DE CONTROLE RAPIDE POUR GROUPES SANGUINS ET LA DETECTION DE LA REPONSE IMMUNO-HEMATOLOGIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **22.09.2004  IT VR20040149**

(43) Date of publication of application:
**20.06.2007  Bulletin 2007/25**

(73) Proprietor: **Sanitaria Scaligera Spa**
**38100 Trento (IT)**

(72) Inventors:
• **ZUCCATO, Alessandro**
**I-37127 Avesa (VR) (IT)**
• **FACCI, Paolo**
**I-16125 Genova (IT)**
• **ALESSANDRINI, Andrea**
**I-61100 Pesaro (IT)**

(74) Representative: **Petraz, Gilberto Luigi et al**
**GLP S.r.l.**
**Piazzale Cavedalis 6/2**
**33100 Udine (IT)**

(56) References cited:
**WO-A-2004/072622     US-A- 5 776 711**

**US-B1- 6 303 390**

• **KRAPF, REINER: "Blutanalytik und Biosensorik mit Schwingquarzen (Dissertation)" INTERNET ARTICLE, [Online] 2001, XP002366180 Retrieved from the Internet: URL:http://w210.ub.uni-tuebingen.de/dbt/vo lltexte/2002/441/pdf/ Dissertation_Krapf.pd f>**
• **TESSIER LOIC ET AL: "AT-cut quartz crystal biosensor for blood assay" PROCEEDINGS OF THE ULTRASONICS INTERNATIONAL CONFERENCE 1993 PUBL BY BUTTERWORTH-HEINEMANN LTD, LONDON, ENGL, 1993, pages 627-630, XP009061215**
• **TESSIER, L., ET AL: "Significance of mass and viscous loads discrimination for an AT-quartz blood group immunosensor" SENSORS AND ACTUATORS, vol. 18-19, 1994, pages 698-703, XP002366182**
• **QUINN, J.G., ET AL: "Detection of blood group antigens utilising immobilised antibodies and surface plasmon resonance" JOURNAL OF IMMUNOLOGICAL METHODS, vol. 206, 1997, pages 87-96, XP002366181**
• **PETR SKLADAL: "Piezoelectric Quartz Crystal sensors Applied for Bioanalytical Assays and Characterization of Affinity Interactions" JOURNAL OF THE BRAZILIAN CHEMICAL SOCIETY, vol. 14, no. 4, 2003, pages 491-502, XP002366183**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**TECHNICAL FIELD**

This invention concerns a rapid monitoring system for blood groups and more in general for detecting immunohematological reactions by means of a detection device named quartz crystal microbalance (QCM).

More specifically, the QCM is used according to this invention for rapid monitoring of blood groups by performing direct and indirect tests.

For direct grouping, IgM type antibodies are selectively immobilized on the surface of the electrodes of an appropriately functionalized QCM transducer, starting from appropriate antiserum, so that red blood cells with the corresponding antigens on their surface can be specifically recognised.

For indirect grouping, the IgM antibodies present in the plasma are captured by the surface of the electrode which is functionalized to give it high specificity for IgM antibodies; the antibody status is then tested by exposure to blood tests.

Optimization of the accuracy of the antibody reaction by means of thermoregulation is a fundamentally important aspect for the success of the test. The method described below makes it possible to carry out tests very quickly (a few seconds for each test), cheaply and automatically.

This invention can be used in the field of diagnostic instruments and in particular in the sector of immunohematological reaction detectors.

**BACKGROUND ART**

It is known that immunodiagnostic methods for blood grouping are currently based on three different functioning principles: hemoagglutination, antibody-antigen bond, genetic analysis.

Hemoagglutination is the oldest method, dating back to the time of the first blood tests, when the donor's and the recipient's blood were simply placed in contact on a glass slide to check for the presence or absence of the agglutination reaction that characterizes incompatibility between the two fluids: if the antibodies in the recipient's serum do not recognize the donor's red blood cells because they belong to a different blood phenotype, they attack them, causing a kind of coagulation (agglutination). Modern tests use monoclonal antibodies specifically created in the laboratory for a selective action against the various blood groups (direct method) or test red blood cells of a known group placed in contact with the patient's serum (indirect method), but the concept does not change.

The tests can be performed in liquid phase (the traditional manual method) or gel phase. The top of the range in this last category is the gel-test system patented by the Swiss company DiaMed. This method involves the use of special cards on which, on top of a layer of gel or glass microspheres, a dose of specific antiserum (anti-A, anti-B, anti-AB, and anti-D) has been applied in the factory.

The technician performing the test places the red blood cells obtained by centrifuging the patient's blood in each of the wells. The card is then centrifuged. If agglutination occurs, the size of the agglutinins does not allow them to pass through the gel. Non-agglutinated red blood cells, on the other hand, pass through the gel unaffected and are deposited at the bottom of the microtube.

By examining the card with the naked eye or with an automatic system, it is therefore easy to distinguish whether or not agglutination has occurred.

A similar card, but filled only with gel (no antiserum), is used for the indirect method: the technician adds test red blood cells of a known group and patient serum on top of the gel before centrifugation. The results are read according to the same logic as above.

The techniques based on hemoagglutination have the main disadvantage of requiring a sequence of controlled-temperature incubations, agitation, resuspension and centrifugation, reducing productivity and also requiring a certain number of devices in order to carry out the test. With respect to other techniques, there is also a certain lack of sensitivity.

A further means of blood group recognition is represented by the tests that exploit the antigen-antibody reaction. These tests used "marked" (i.e. bound) antibodies or antigens with an easily recognizable substance, using this substance to detect the amount of marked antibody or antigen that had bound with the surface antigens present on the red blood cells or antibodies present in the serum. All these methods are performed inside the microwell plates, the bottom of which is covered with unmarked antibodies specific for each test.

There are three main families of immunohematological methods: immunocompetitive assays, immunometric assays and immunoabsorbent assays.

The class of immunocompetitive assays (EIA/RIA), the first in the immunohematology family from a historical point of view, foresees the use of a microplate divided into a number of wells, at the bottom of which antibodies specific for the antigen to be detected are chemically bound. The sample to be analyzed and a very precise amount of the same antigen, but "marked" by replacing one of its component atoms with a radioactive isotope - usually tritium, iodine 125 or carbon 14 (RIA, Radioactive Immuno Assay) or by chemically binding it to an enzyme, typically alkaline phosphatase (EIA, Enzymatic Immuno Assay), are placed inside the well.

The greater the amount of "natural" antigen in the sample, the lesser the amount of marked antigen that will be bound by the antibodies fixed to the bottom of the well. After washing to eliminate anything not bound to these antibodies, the binding percentage is assessed in the case of RIA by a radiation counter. In the other cases, again after washing, a substance that reacts with the marker enzyme is placed in the well, causing a change in colour (usually tending towards yellow) or creating a fluorescent or phosphorescent substance.

The colour change can be assessed visually or by photocells, while fluorescence or phosphorescence are evaluated by automatic devices equipped with light amplifiers, which give a greater precision in assessing the results.

The RIAs were the first immunohematological tests, dating back to the 70s. However, the lability of the marked molecules and the strict regulation of the radioactive isotopes decreed the obsolescence of these tests some time ago in favour of EIAs which are still widely used.

According to the immunometric assay method (Sandwich), the sample containing the antigen to be tested is placed in the well, where it is bound by the antibodies bound to the bottom of the well. An antibody marked with an enzyme (for example alkaline phosphatase) is then added. This antibody binds to the immobilized antigen. Washing is carried out to remove any unbound substance, and a reagent is added which reacts with the enzyme and, in this case, causes a change in colour, phosphorescence or fluorescence, this time directly (rather than inversely) proportional to the amount of antigen present in the fluid being tested.

This change in colour, phosphorescence or fluorescence is assessed in the same way as above. This type of test has very rapid kinetics and a greater sensitivity compared to the EIAs.

The immunoabsorbent assay method, named ELISA (Enzyme-Linked Immunosorbent Assay), is nowadays widely used not only for blood grouping but also, and above all, because of the high specificity and very high sensitivity, for determining the presence of antibodies that respond to pathogens (e.g. HIV, HCV). Unlike the previous tests, the substance bound to the bottom of the microplate does not consist of antibodies but is a solid phase consisting of an inactivated virus or of synthesis peptide fragments the same as those present on the surface of the antigen whose presence is being assessed - in the case of blood grouping, the surface antigens of the various red blood cell phenotypes.

The patient's biological fluid is placed in this well: if the patient has been exposed to the virus, the antibodies against the virus will be present in the fluid and will bind to the solid phase. In the case of blood grouping, the patient's serum will instead contain antibodies against the other red blood cell phenotypes, which will attack the surface antigens present at the bottom of the well. The plate is then washed to remove any non-adsorbed substance and an anti IgG/IgM antibody, i.e. a non-human marked antibody specifically targeted against human immunoglobulins, is added to the well, binding to the patient's antibodies bound in turn to the solid phase. Further washing eliminates the unbound substances: the well now contains only the solid phase, to which the patient's antibodies are bound and which are covered with the marked anti-IgG/IgM antibodies. An appropriate substrate is then added, reacting with the marker enzyme (e.g. 5-aminosalicylic acid or O-phenyldiamine in the case of peroxidase; 4-nitrophenylphosphate in the case of alkaline phosphatase), allowing the results to be read.

The ELISA tests are characterized by a very high sensitivity, since a large quantity of marked antibodies binds to a small amount of patient antibody. In blood grouping, these tests are therefore mainly used to detect irregular antibodies, as well as for screening against infections that can be transmitted via the blood (for example, the various forms of hepatitis).

There are numerous devices on the market that can perform analyses with all the above-mentioned methods - EIA, Sandwich and ELISA-for example the various Immucor Galileo, Prism and many other devices.

Among the main disadvantages of these techniques, however, is the need to use reagents (marked antibodies/antigens) that are very expensive and the need for great precision in dosing the sample and the reagents. All the techniques also require at least one washing phase, if not two separate washing phases as in the case of the ELISA technique, to eliminate anything which is not part of the antigen-antibody bond. This causes microfluid or manipulation problems, as well as making the test less portable for use in the field.

The methods based on genetic analysis are very recent techniques, based on the analysis of the points of the human genome relative to the encoding of the superficial peptides of the red blood cells - for example the point of chromosome 9 which identifies the ABO blood group, but also the points which encode other surface peptides, such as the Rh factor, or the rare phenotypes. This is a complex process, involving DNA amplification by means of processes such as polymerase chain reaction (PCR) and numerous other processes.

It should be noted that the use of genetic tests presents notable advantages, not least a heuristic approach to diagnostics, i.e. a more overall vision compared to a single test, and the availability of DNA suitable for analysis in tissues other than the blood, such as for example epithelial tissues.

Genetic diagnostics is still in the early stages of development, the methods being mainly experimental at present, applied more in the sector of forensic medicine than in the transfusion field.

This sector is, however, rapidly expanding and many companies are investing considerable resources. Genetic tests are already raising ethical questions linked to the possible incorrect use of the information gathered. Even if any legal impediments related to these questions are ignored, experts nevertheless foresee that it will be at least ten years before reliable and affordable genetic tests are available.

## DESCRIPTION OF THE INVENTION

This invention proposes to provide a system for monitoring immunohematological reactions that is able to eliminate or at least reduce the drawbacks described above.

The invention also proposes to provide a system for monitoring immunohematological reactions based on the use of quartz crystal microbalances (QCM), which are sensitive transducers that offer the possibility of studying immunological reactions without the need to mark molecules. This technique can be used both to measure reaction kinetics and the concentration of various (bio)analytes in solution.

This is achieved by means of a system for monitoring immunohematological reactions whose features are described in the main claim.

The dependent claims of the solution in question describe advantageous embodiments of the invention.

The main advantages of this solution concern the possibility of implementing techniques for rapid blood group determination, with direct and indirect analysis (red blood cells), and hepatitis screening, by use of the QCM.

The quartz crystal microbalance (QCM) is a device that can measure very small variations in mass (down to fractions of a nanogram). The extreme sensitivity to the mass is due to the use of small piezoelectric crystals oscillated at resonance frequency. This resonance frequency greatly depends on the mass present on the surface of the quartz and, by monitoring the trend of the resonance frequency, this makes it possible to follow the adsorption of more complex molecules or structures, for example cells, on the surface of the quartz. The equations describing the relationship between resonance frequency and adsorbed mass for an AT-cut crystal establish a direct proportion between frequency variation $\Delta f$ and mass variation $\Delta m$:

$$\Delta f = - C \, \Delta m$$

The constant C represents the calibration coefficient and can be determined by placing known masses on the surface of the crystal.

A driver connected to a quartz crystal oscillator guides the transducer to oscillate at the correct resonance frequency. The output signal of the driver is sent to a frequency meter which measures the frequency. The entire system can be guided by a computer that can records the temporal variations of the resonance frequency.

The microbalance can function even when one of the two surfaces covered by the electrodes is immersed in liquid. In this last case the previous relationship between adsorbed mass and frequency variation is no longer valid and must be replaced by more complex equations that take into account other physical parameters of the liquid layer adsorbed on the surface (density, share viscosity). After removing the microbalance from the liquid it is however always possible to measure the resonance frequency and recover information on the adsorbed mass.

By functionalizing one of the two faces of the crystal with certain molecules it is possible to follow in-situ or to determine ex-situ the presence of specific recognition events between the molecules immobilized on the surface of the quartz and other molecules present in solution.

The quartz microbalance has been used to study various protein-protein interactions, including in particular the antibody-antigen interaction.

In general the use of the QCM in these cases is designed to exploit the sensoristic properties of the device. The antibody being tested is immobilized with appropriate techniques on one of the two electrodes of the microbalance in such a way as to preserve its biological functionality.

The presence of the corresponding antigen in solution is determined by monitoring the variations of resonance frequency of the crystal following any specific bond between the antibody and the antigen and consequent increase in actual mass on the electrode.

The microbalance technique has also been applied in the study of more complex systems such as viruses, bacteriophages and cells. In the first two cases the approach foresees the immobilization of specific antibodies on the microbalance electrode and detection of the specific bond between the virus or bacteriophage and the antibody.

When studying cells, on the other hand, interest is focussed on the viscoelastic type modifications that a cell population adhered to the balance electrode undergoes following pharmacological treatment. This is based on the ability of the microbalance to detect not only variations in adhered mass, but also variations in the viscoelastic properties of the mass.

Document "Blutanalitytik und Biosensorik mit Schwingquartzen (Dissertation)" of Reiner Krapf, 2001, Internet Article retrievable at the following URL: http://w210.ub.uni-tuebingen.de/dbt/volltexte/2002/441/ discloses a system for immunological measurements comprising a 10 MHz quartz crystal microbalance coupled with a flow injection system. The measurement is based on the detection of the resonance frequency change on the AT-cut qurtz crystal resulting from the mass change on the quartz surface. This quartz is functionalized using antibodies and is used for ABO blood grouping and for determination of the rhesus factor. The antibody immobilisation is performed by physisorption, by coupling via alkanethiols and by coupling with Protein A, and the functionalisation of the quartz by IgM antibodies is performed via a layer of Protein A. The binding of the IgM antibodies and IgM on a protein-A coated quartz is observed via the resonance frequency change.

## DESCRIPTION OF THE DRAWINGS

Other features and advantages of the invention will become evident on reading the following description of one embodiment of the invention, given as a non-binding example, with the help of the accompanying drawings in which:

- figure 1 represents the setup diagram of the quartz microbalance;
- figure 2 represents a schematic view of the immobilization of the antibodies on the balance electrode;
- figure 3 shows an atomic force microscope image of IgM molecules immobilized on a surface exhibiting SH groups;
- figure 4 represents optical microscope analysis of red blood cell immobilization;

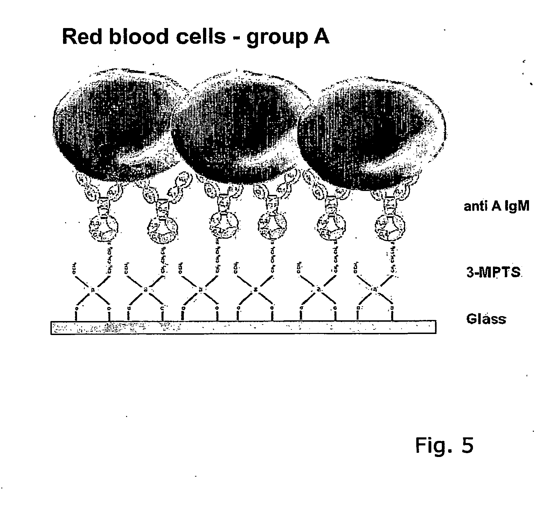

- figure 5 shows red blood cell immobilization on a functionalized glass slide;
- figure 6 represents QCM frequency variation monitoring;
- figure 7 is the first table with the series of tests on the QCM for direct grouping;
- figure 8 is the second table with the series of tests on the QCM for indirect grouping;
- figure 9 shows the wiring diagram of a possible form of driver for the QCM.

## DESCRIPTION OF ONE EMBODIMENT OF THE INVENTION

The quartz microbalance (QCM) is a device that can measure very small variations in mass (down to fractions of a nanogram).

The extreme sensitivity to mass is due to the use of small piezoelectric crystals oscillated at their resonance frequency.

This resonance frequency greatly depends on the mass present on the surface of the quartz and, by monitoring the trend of the resonance frequency, this makes it possible to follow the adsorption of more complex molecules or structures, for example cells, on the surface of the quartz. The equations describing the relationship between resonance frequency and adsorbed mass for an AT-cut crystal establish a direct proportion between frequency variation Δf and mass variation Δm:

$$\Delta f = - C \, \Delta m$$

The constant C represents the calibration coefficient and can be determined by placing known masses on the surface of the crystal.

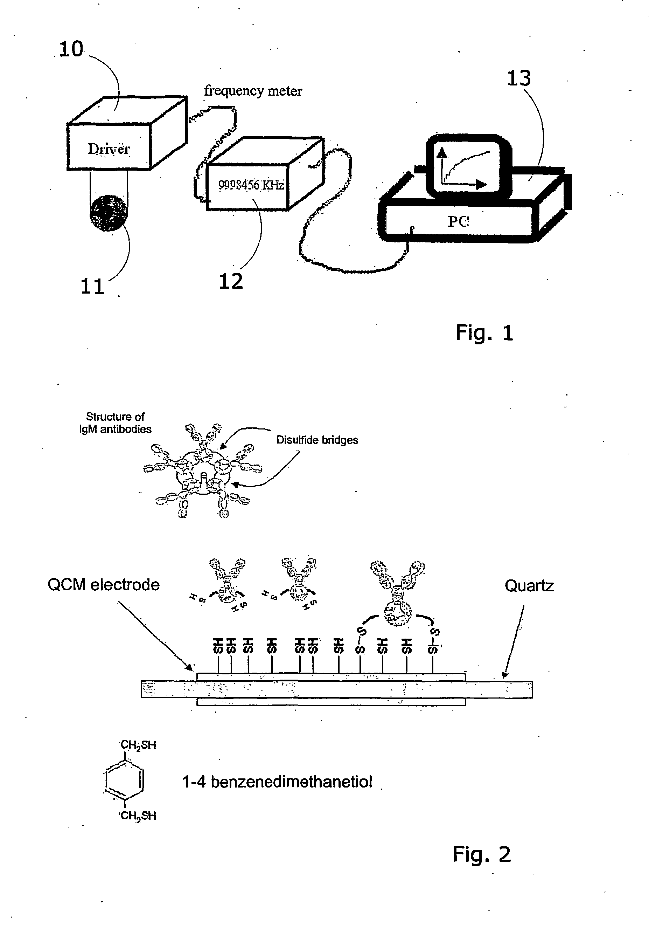

Figure 1 shows a diagram of the experimental setup used.

A driver 10 connected to a quartz crystal oscillator 11 guides the transducer to oscillate at the correct resonance frequency.

The output signal of the driver 10 is sent to a frequency meter 12 which measures the frequency. The entire system can be guided by a computer 13 that can record the temporal variations of the resonance frequency.

The microbalance can function even when one of the two surfaces covered by the electrodes is immersed in liquid. In this last case the previous relationship between adsorbed mass and frequency variation is no longer valid and must be replaced by more complex equations that take into account other physical parameters of the liquid layer adsorbed on the surface (density, share viscosity). After removing the microbalance from the liquid it is however always possible to measure the resonance frequency and recover information on the adsorbed mass.

By functionalizing one of the two faces of the crystal with certain molecules it is possible to follow *in-situ* or to determine *ex-situ* the presence of specific recognition events between the molecules immobilized on the surface of the quartz and other molecules present in solution.

The quartz microbalance has been used to study various protein-protein interactions, including in particular the antibody-antigen interaction.

In general the use of the QCM in these cases is designed to exploit the sensoristic properties of the device.

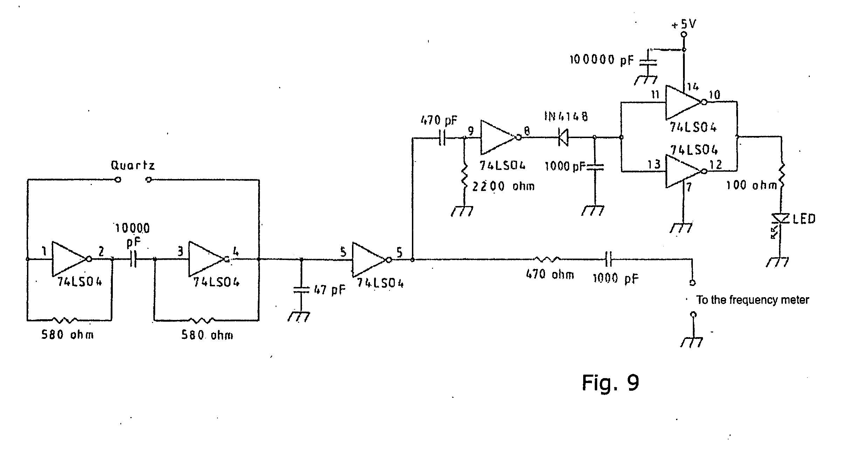

Figure 9 shows the wiring diagram of the possible form of a driver for the QCM.

The antibody being tested is immobilized with appropriate techniques on one of the two electrodes of the microbalance in such a way as to preserve its biological functionality.

The presence of the corresponding antigen in solution is determined by monitoring the variations of resonance frequency of the crystal following any specific bond between the antibody and the antigen and consequent increase in actual mass on the electrode.

The microbalance technique is also applied in the study of more complex systems such as viruses, bacteriophages and cells. In the first two cases the approach foresees the immobilization of specific antibodies on the microbalance electrode and detection of the specific bond between the virus or bacteriophage and the antibody.

When studying cells, on the other hand, interest is focussed on the viscoelastic type modifications that a cell population adhered to the balance electrode undergoes following pharmacological treatment. This is based on the ability of the microbalance to detect not only variations in adhered mass, but also variations in the viscoelastic properties of the mass.

The quartz microbalance has also been used to determine agglutination phenomena in solution between spheres of latex covered with antibodies induced by the presence of specific proteins. These agglutination phenomena cause a notable variation in the viscosity of the liquid in which the balance is immersed and this variation in viscosity is reflected in a variation in the resonance frequency.

The antibodies of the immunohematological system, in particular the IgMs, present in appropriate animal antiserum, are immobilized on the surface of an electrode of a quartz microbalance (resonance frequency around 10 MHz) to determine the specific recognition of red blood cells provided with complementary antigens on their cellular membrane.

The description reported below considers the ABO system, but the method and the technique described can also be applied to other systems.

Immobilization of the IgM type antibodies on the surface of the QCM transducers is achieved by first forming a self-assembled layer of molecules able to selectively bind the IgMs present in sheep antisera.

In the case in question, the self-assembled layer consists of molecules of 1-4 benzenedimethanetiol, which bind to the silver of the electrode present on the surface of the QCM transducer by using one of their two thiols and at the same time exposing the other thiol to form a covalent bond capable of immobilizing the antibody.

The bond with the antibody occurs by means of the thiol-disulfide exchange reaction. The disulfide bridges which the IgM are rich in are reduced close to the thiolated surfaces and the free thiols in turn form disulfide bridges with the thiols exposed by the self-assembled layer (figure 2).

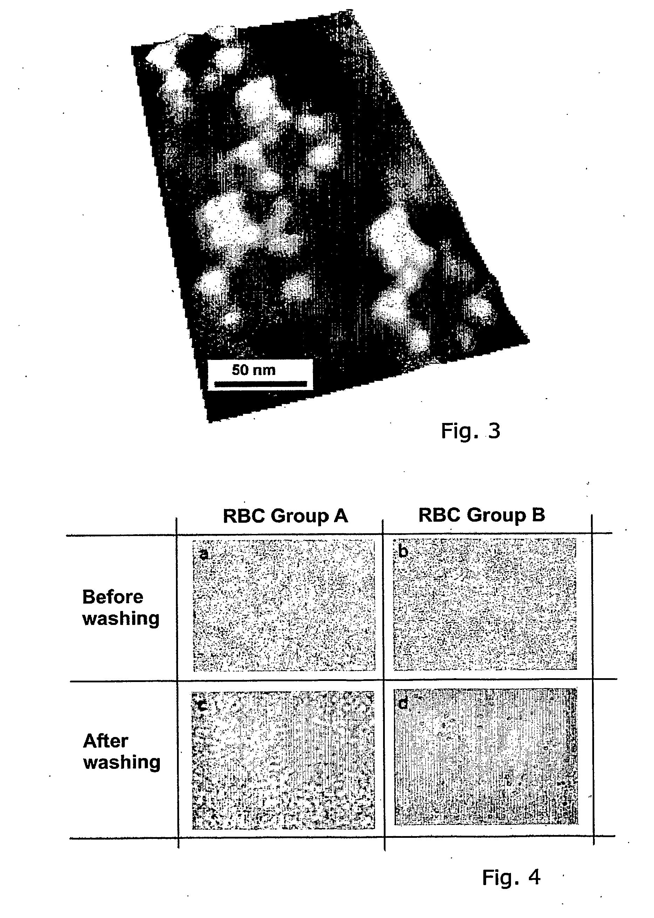

The actual immobilization of the antibodies on a surface exhibiting SH groups was verified with atomic force microscopy (AFM), which clearly shows the immobilization and the high specificity of the process, figure 3.

To check that the immobilized antibodies maintain their biological functionality, an optical microscopy study was performed on functionalized glass slides to expose the SH type functional groups. The results are shown in figure 3.

Two slides were functionalized with silanes (3-MPTS, 3-Mecaptopropyltrimethoxisilane) able to covalently bind on one side to the slide and to expose SH groups. It is important to point out that the chemistry of the first functionalization layer on the surface of the slide should be considered similar to the chemistry performed on the electrode of the quartz balance.

Even if the bond between the molecules and the solid surface is different, the exposed functional group is the same. The two slides were then exposed to the serum containing anti A type IgMs.

One of the two slides was then incubated with group A whole blood (figure 4a) while the remaining slide was incubated with group B whole blood (figure 4b). Figures 4a and 4b show slides incubated with blood before washing.

Figures 4c and 4d are the images of the slides in figures 4a and 4b respectively, after washing in saline solution. It is clear that only in the case of blood group A has a specific bond occurred between the antibodies present on the surface and the antigens present on the surface of the red blood cells (figure 4). The optical microscopy analysis confirms that the immobilized antibodies are biologically active, maintaining specificity for the corresponding antigens.

For monitoring of the specific recognition with the quartz microbalance, each functionalization process is checked by measuring the resonance frequency value of the crystal ex-situ and comparing it with the corresponding value of the previous phase.

The first value acquired corresponds to the resonance frequency value of the quartz without any treatment. The quartz, equipped with silver electrodes, is functionalized by exposure to a 1 mg/ml solution of 1-4 benzenedimethanetiol in toluene for 2 minutes followed by washing with abundant toluene to remove the molecules that have not bound to the electrode. The resonance frequency of the functionalized quartz is then measured. A reduction in frequency confirms functionalization of the electrode. The quartz is then exposed to the serum containing the IgM antibodies for 5 minutes and rinsed with saline solution. The resonance frequency of the quartz is measured again. A further reduction in frequency confirms immobilization of the antibodies. In the final stage, the quartz functionalized with the antibodies is exposed for 5 minutes to the whole blood undergoing direct grouping, appropriately diluted in saline solution up to a typical concentration of $3 \cdot 10^4$ rbc/$\mu$l.

The temperature at which the system should be maintained during incubation is an absolutely critical parameter for optimization of the reaction accuracy.

The surface of the quartz is then washed, using an appropriate washing cell which avoids direct flow of the solution on the surface and prevents problems of surface voltage connected with the repeated crossing of the surface of the solution by the electrode.

Repeated checks have shown that the temperature interval of the IgM-antigen reaction is 4° - 22°C with an optimum value of 10°$\pm$3°C. This temperature must also be maintained during the subsequent washing stage of the non-specific adsorption compounds.

The tests carried out-used blood grouping performed with traditional techniques on the same sample as a reference. The reading of the final resonance frequency of the quartz makes it possible to establish whether specific recognition has occurred between the antibodies immobilized on the surface and the membrane antigens of the red blood cells.

A significant reduction in resonance frequency (more than a thousand Hz) is considered significant evidence that the blood group of the blood to which the crystal has been exposed specifically recognizes the antibodies on the quartz, In the case of anti-A antibodies, the specific recognition will take place for group A red blood cells.

In the case of anti-A antibodies and group B blood, the expected variation in frequency is less than a thousand Hz.

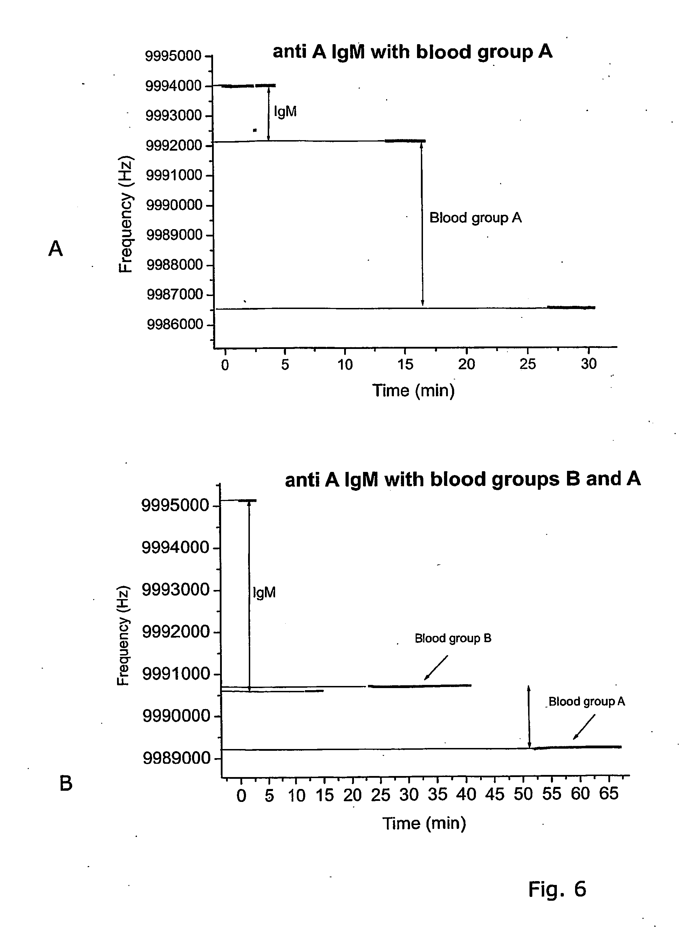

Figure 6 shows two examples of resonance frequency variation, starting from the immobilization phase of the antibodies, in both cases anti-A, and known blood group A (fig. 6a) and B and then A (fig. 6b). In both cases there is an evident decrease in resonance frequency following immobilization of the antibodies, while the decrease in resonance frequency following exposure to blood is present only in the case of group A blood, for which there is specific recognition of the immobilized antibodies.

In figure 6b, after being exposed to group B blood, the quartz is exposed to group A blood, for which specific recognition is expected. In fact, following exposure to group A blood, a significant decrease in resonance frequency can be measured once again.

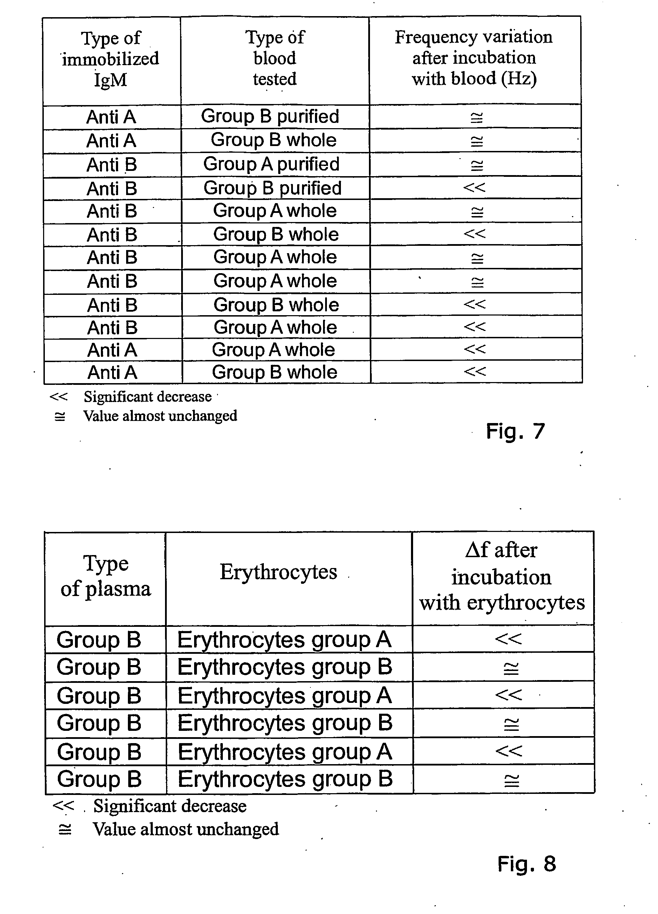

The table in fig. 7 shows an example of results obtained with various combinations immobilized antibodies and different blood groups. The variation in frequency in the final stage is indicated with the symbol << when significant (above the threshold of 1000 Hz) and with the symbole $\cong$ when the variation is below the threshold of 1000 Hz.

Up to this point, the method described allows direct blood group determination, but a blood group test can only be considered valid if indirect tests are also carried out.

Indirect tests mean determination of the presence, in the plasma, of the natural antibodies relative to the particular blood group. For indirect determination of the blood group, a procedure similar to the case of direct grouping was used (in particular it is fundamentally important that the IgM-antigen reaction and the subsequent washing for removal of the aspecific adsorption compounds take place at a temperature in the range of 4-22°C, with optimum performance at $10° \pm 3°C$), the difference being that the blood group of the red blood cells used is known (test erythrocytes), the aim being to determine the type of IgM antibodies present in the plasma, which is separated from the whole blood by centrifugation.

The quartz is functionalized in the same way as the previous case, using 1-4 benzenedimethanetiol. The functionalized quartz is exposed to the plasma for 15 minutes to capture and immobilize the IgM antibodies present in the plasma. In this case too, measurement of the resonance frequency of the quartz after each functionalization phase makes it possible to check that immobilization has in fact taken place.

After exposure to the plasma, the quartz is exposed to the test red blood cells. In the case of plasma from blood whose direct blood group has been identified as type A, the IgM antibodies which should have been immobilized by the functionalized quartz surface are the anti-B type, so that the test red blood cells that should be immobilized are group B.

The table in figure 8 shows a representative set of results obtained for indirect grouping. The variation in the final resonance frequency is shown in the table according to the same criteria used for the direct grouping table. As can be seen, capture of the test red blood cells of a certain group takes place only if the quartz was exposed to the plasma of blood containing complementary IgM antibodies.

With a similar approach and data, the functioning of the test was also checked for groups AB and 0 and for determining the Rh factor.

The use of a multiparametric QCM system integrated on a single crystal that can accommodate several sensors working in parallel or with multiplexer logic is envisaged.

These sensors are produced from a single AT-cut quartz crystal and are defined by means of lithographic techniques.

According to this system, each sensor can be addressed independently of the others and can be made specific for a certain antigen/antibody/antigenic determinant by means of selective functionalization, also assisted by microfluidics applied on the chip itself or on disposable polymer supports.

This system is therefore able to perform a complete series of analyses (for example direct grouping + hepatitis markers) by means of a single exposure to the biological fluid being tested, speeding up the time necessary for the tests and making the approach substantially easier and more automated.

The invention is described above with reference to a preferred embodiment. It is nevertheless clear that the invention is susceptible to numerous variations that lie within the framework of technical equivalents.

**Claims**

1. A system for monitoring blood groups and for detecting immunohematological reactions, comprising a detection device consisting of a quartz crystal microbalance (QCM), whereby the equations describing the relationship between the resonance frequency and the adsorbed mass for an AT-cut quartz crystal establish a direct proportion between frequency variation $\Delta f$ and mass variation $\Delta m$: $\Delta f = - C \Delta m$, where the constant C represents the calibration coefficient and can be determined by placing a known mass on the surface of the quartz crystal, the system further comprising a driver (10) connected to a quartz crystal oscillator (11) consisting of an AT-cut quartz crystal equipped with metal electrodes facing each other on two sides, with its own frequency of around several MHz, which guides the QCM to oscillate at the resonance frequency, whereby the output signal of the driver (10) is sent to a frequency meter (12) which measures the frequency, whereby one of the two sides of the quartz crystal is functionalized by means of predetermined molecules in order to follow *in-situ* or to determine *ex-situ* the presence of specific recognition events between the molecules immobilized on the surface of the quartz and other molecules present in solution, said system allowing detection of antibodies, where the antibody being tested is immobilized on one of the two microbalance electrodes in such a way as to preserve the biological functionality, whereby the presence of the corresponding antigen in solution is determined by monitoring the variations in resonance frequency of the crystal following any bond between the antibody and antigen and a consequent increase in actual mass on the electrode, said system being **characterised in that** the immobilization of IgM antibodies on the surface of the QCM transducer is achieved by prior formation of a self-assembled layer of molecules which can selectively bind the IgMs present in the antisera, whereby the functionalization technique exploits molecules which can bind the QCM electrode metal and expose -SH moieties which can selectively bind IgMs from an antiserum.

2. A system according to claim 1 **characterised in that** the reaction interval for IgM-antigen antibodies is 4-22°C, with an optimum value of 10°±3°C.

3. A method for performing indirect grouping, i.e. quantification of the presence of antibodies in plasma, using a system according to any one of the preceding claims, **characterised in that** the QCM electrode metal is first functionalized, followed by the exposure of the functionalized QCM to a serum under test in order to capture IgMs present therein, and by a further incubation of the QCM with test RBC (red blood cells of known group) enabling to reveal the presence of given IgMs in the serum under test, as previously captured by the surface.

## Patentansprüche

1. System zur Überwachung von Blutgruppen und zum Nachweis von immunhämatologischen Reaktionen, mit einer Detektionseinrichtung, bestehend aus einer Quarzkristallmikrowaage (QCM), wobei die Gleichungen, die die Beziehung zwischen der Resonanzfrequenz und der adsorbierten Masse für einen in einer AT-Richtung geschnittenen Quarzkristall beschreiben, eine direkte Proportionalität zwischen der Frequenzänderung $\Delta f$ und der Massenänderung $\Delta m$ : $\Delta f = - C \, \Delta m$ begründen, wobei die Konstante C den Kalibrierungskoeffizienten darstellt und bestimmt werden kann, indem eine bekannte Masse auf der Oberfläche des Quarzkristalls plaziert wird, sowie das System weiterhin umfasst, einen Treiber (10), der mit einem Quarzkristalloszillator (11) verbunden ist, bestehend aus einem in einer AT-Richtung geschnittenen Quarzkristall, der mit Metallelektroden ausgestattet ist, die sich an zwei Seiten gegenüberliegen, und der seine eigene Frequenz von etwa einigen MHz hat, und der die QCM anregt, damit diese bei der Resonanzfrequenz oszilliert, wobei das Ausgangssignal des Treibers (10) zu einer Frequenzmesseinrichtung (12) geschickt wird, die die Frequenz misst, wobei eine der beiden Seiten des Quarzkristalls durch bestimmte Moleküle funktionalisiert ist, um einer in-situ oder eine ex-situ Anwesenheit der spezifischen Erkennungsvorfälle zwischen den Molekülen zu folgen oder diese zu bestimmen, die an der Oberfläche des Quarzes immobilisiert sind und anderen Molekülen, die in der Lösung vorhanden sind, und wobei das System die Erkennung von Antikörpern erlaubt, wobei der Antikörper, der zu testen ist, an einer der beiden Mikrowaagenelektroden immobilisiert ist, derart, dass dessen biologische Funktionalität bewahrt ist, wobei das Vorhandensein des korrespondierenden Antigens in der Lösung festgestellt wird, indem die Variationen der Resonanzfrequenz des Kristalls überwacht werden, welche jeglichem Anhaften zwischen dem Antikörper und dem Antigen folgen sowie einer darauf folgenden Erhöhung der aktuellen Masse an der Elektrode, wobei das System **dadurch gekennzeichnet ist, dass** die Immobilisierung von IgM-Antikörpern auf der Oberfläche des QCM-Meßwandlers durch die vorherige Ausbildung einer selbstanordnenden Schicht von Molekülen erreicht wird, die die IgMs selektiv binden können, die in dem Antiserum vorhanden sind, während die Funktionalisierungstechnik Moleküle verwertet, die das QCM-Elektrodenmetall binden und die -SH-Hälften freistellen, die selektiv IgMs aus dem Antiserum binden.

2. System nach Anspruch 1, **dadurch gekennzeichnet, dass** das Reaktionsintervall für die IgM-Antigen-Antikörper 4-22 °C beträgt, mit einem optimalen Wert von 10°±3°C.

3. Verfahren zur Durchführung des indirekten Gruppierens, d.h. der Quantifizierung der Anwesenheit von Antikörpern in einem Plasma, unter Verwendung eines Systems nach einem der vorherigen Ansprüche, **dadurch gekennzeichnet, dass** die QCM-Elektrode zuerst funktionalisiert wird, gefolgt von der Aussetzung der funktionalisierten QCM in einem Serum im Test, um IgMs zu fangen, die darin vorhanden sind, sowie durch eine weitere Inkubation des QCM mit Test-RBC (rote Blutzellen einer bekannten Gruppe), wodurch es ermöglicht wird, die Anwesenheit von bestimmten IgMs in dem Serum unter Test aufzuzeigen, die vorher von der Oberfläche eingefangen worden sind.

## Revendications

1. Système de contrôle de groupes sanguins et de détection de réactions immuno-hématologiques, comprenant un dispositif de détection constitué d'une microbalance à cristal de quartz (ou MCQ), dans lequel les équations décrivant la relation entre la fréquence de résonance et la masse adsorbée pour un cristal de quartz de coupe AT établissent une proportion directe entre la variation de fréquence Δf et la variation de masse Δm : Δf = -C Δm, où la constante C représente le coefficient d'étalonnage et peut être déterminée en plaçant une masse connue sur la surface du cristal de quartz, le système comprenant en outre un pilote (10) raccordé à un oscillateur à cristal de quartz (11) constitué d'un cristal de quartz de coupe AT équipé d'électrodes métalliques se faisant face sur deux côtés, avec sa fréquence propre de plusieurs MHz environ, lequel pilote commande la MCQ d'osciller à la fréquence de résonance de sorte que le signal de sortie du pilote est envoyé à un fréquencemètre (23) mesurant la fréquence, dans lequel un des deux côtés du cristal de quartz est fonctionnalisé au moyen de molécules prédéterminées afin de suivre *in situ* ou de déterminer *ex-situ* la présence d'événements de reconnaissance spécifiques entre les molécules immobilisées sur la surface du quartz et d'autres molécules présentes en solution, ledit système permettant de détecter des anticorps, où l'anticorps devant être testé est immobilisé sur l'une des deux électrodes de la microbalance de manière à conserver la fonctionnalité biologique, dans lequel la présence de l'antigène correspondant en solution est déterminé en contrôlant les variations de la fréquence de résonance du cristal suivant toute liaison entre l'anticorps et un antigène et une augmentation importante de la masse réelle sur l'électrode, ledit système étant **caractérisé en ce que** l'immobilisation d'anticorps IgM sur la surface du transducteur MCQ est obtenue par la formation préalable d'une couche auto-assemblée de molécules qui peuvent se lier sélectivement aux IgMs présents dans les antisérums, par lequel la technique de fonctionnalisation exploite des molécules qui peuvent se lier au métal d'électrode de la MCQ et exposer des fractions -SH qui peuvent se lier sélectivement à des IgMs provenant d'un antisérum.

2. Système selon la revendication 1, **caractérisé en ce que** l'intervalle de réaction des anticorps IgMs-antigène est 4-22°C, avec une valeur optimale de 10°C ± 3°C.

3. Procédé de réalisation d'un groupement indirect, c'est-à-dire de la quantification de la présence d'anticorps dans un plasma, en utilisant un système selon l'une quelconque des revendications précédentes, **caractérisé en ce que** le métal d'électrodes de la MCQ est en premier lieu fonctionnalisé, suivi de l'exposition de la MCQ fonctionnalisée à un sérum à tester, afin de capturer des IgMs présents à l'intérieur, et d'une incubation supplémentaire de la MCQ avec des globules rouges de test (cellules sanguines rouges d'un groupe connu) permettant de révéler la présence d'IgMs connus dans le sérum à tester, tels qu'ils ont été capturés précédemment par la surface.

Fig. 1

Structure of
IgM antibodies

Disulfide bridges

QCM electrode

Quartz

1-4 benzenedimethanetiol

Fig. 2

Fig. 3

Fig. 4

## Red blood cells - group A

anti A IgM

3-MPTS

Glass

Fig. 5

Fig. 6

| Type of immobilized IgM | Type of blood tested | Frequency variation after incubation with blood (Hz) |
|---|---|---|
| Anti A | Group B purified | $\cong$ |
| Anti A | Group B whole | $\cong$ |
| Anti B | Group A purified | $\cong$ |
| Anti B | Group B purified | << |
| Anti B | Group A whole | $\cong$ |
| Anti B | Group B whole | << |
| Anti B | Group A whole | $\cong$ |
| Anti B | Group A whole | $\cong$ |
| Anti B | Group B whole | << |
| Anti B | Group A whole | << |
| Anti A | Group A whole | << |
| Anti A | Group B whole | << |

<<  Significant decrease
$\cong$  Value almost unchanged

**Fig. 7**

| Type of plasma | Erythrocytes | $\Delta f$ after incubation with erythrocytes |
|---|---|---|
| Group B | Erythrocytes group A | << |
| Group B | Erythrocytes group B | $\cong$ |
| Group B | Erythrocytes group A | << |
| Group B | Erythrocytes group B | $\cong$ |
| Group B | Erythrocytes group A | << |
| Group B | Erythrocytes group B | $\cong$ |

<<  . Significant decrease
$\cong$  Value almost unchanged

**Fig. 8**

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **Reiner Krapf.** *Blutanalitytik und Biosensorik mit Schwingquartzen (Dissertation),* 2001, http://w210.ub.uni-tuebingen.de/dbt/volltexte/2002/441